(19) Europäisches Patentamt
European Patent Office
Office européen des brevets

(11) Numéro de publication: **0 005 680**
**B1**

(12) **FASCICULE DE BREVET EUROPEEN**

(45) Date de publication du fascicule du brevet: **12.05.82**

(51) Int. Cl.³: **C 07 C 69/14,** C 07 C 67/04

(21) Numéro de dépôt: **79420024.6**

(22) Date de dépôt: **10.05.79**

(54) **Procédé de préparation de l'acétate d'éthyle.**

(30) Priorité: **17.05.78 FR 7815355**

(43) Date de publication de la demande:
**28.11.79 Bulletin 79/24**

(45) Mention de la délivrance du brevet:
**12.05.82 Bulletin 82/19**

(84) Etats contractants désignés:
**BE CH DE GB IT LU NL SE**

(56) Documents cités:
**US - A - 2 858 311**
**US - A - 3 474 131**

(73) Titulaire: **RHONE-POULENC INDUSTRIES**
**22 Avenue Montaigne**
**F-75008 Paris (FR)**

(72) Inventeur: **Gruffaz, Max**
**6, Avenue du Général de Gaulle**
**F-69350-La Mulatiere (FR)**
Inventeur: **Micaelli, Odile**
**11, rue Montvert**
**F-69008 - Lyon (FR)**

(74) Mandataire: **Rioufrays, Roger et al,**
**RHONE-POULENC RECHERCHES Centre de Recherches de Saint-Fons Service Brevets B.P. 62**
**F-69190 Saint-Fons (FR)**

**EP 0 005 680 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Procédé de préparation de l'acétate d'éthyle

La présente invention concerne un procédé de préparation de l'acétate d'éthyle à partir d'acide acétique et d'éthylène. L'invention a plus particulièrement pour objet la préparation de l'acétate d'éthyle par réaction de l'acide acétique sur l'éthylène, en phase vapeur, en présence d'un type particulier de résines échangeuses d'ions.

Il est bien connu que l'on peut faire réagir l'acide acétique sur l'éthylène en présence de catalyseurs acides pour obtenir de l'acétate d'éthyle. Un certain nombre de catalyseurs et des conditions opefatoires ont été proposées dans la littérature. Par exemple, des auteurs ont testé divers catalyseurs pour réaliser la réaction envisagée, en phase vapeur. (Cf. Y. MURAKAMI, T. MATTORI et H. UCHIDA, Kogyo Kagaku zasshi, *72*, pages 1945—1948, 1969). Ces auteurs ont, notamment, comparé l'activité catalytique des mélanges d'acide silicotungstique et de gel silice, ou d'acide phosphorique et de kieselguhr, avec celle de résines échangeuses d'ions telles l'AMBERLITE IR 120 B et l'AMBERLYST 15, la première étant de type gélatinoïde, la seconde de type macroporeux, toutes deux étant commercialisées par la Société ROHM et HAAS. Ces auteurs ont conclu que l'acide phosphorique sur kieselguhr, ainsi que l'AMBERLITE IR 120 B, n'ont pratiquement acune activité catalytique dans la réaction envisagée. Ces auteurs ont, d'autre part, mis en évidence que, si les catalyseurs à base d'acide silicotungstique ont une activité initiale appréciable à des températures relativement élevées, ils sont désactivés au bout de quelques heures de fonctionnement. Leur efficacité dans la réaction envisagée est pratiquement nulle à des températures inférieures à 150°C; à hautes températures ces catalyseurs favorisent la formation d'acétone.

Ces auteurs ont montré que, si l'AMBERLYST 15 est le catalyseur le plus efficace dans la synthèse en cause, ce catalyseur de désactive également au bout de quelques heures à la température requise par la réaction.

Les catalyseurs du premier type ne conviennent donc pas pour la mise en oeuvre de la réaction, en phase gazeuse, en raison de leur faible activité. Quant à l'utilisation des résines envisagées dans les travaux antérieurs, elle a échoué à cause de l'instabilité et de la désactivation desdites résines, à la température requise pour un bon déroulement de la réaction. En conclusion, les propositions de l'art antérieur pour préparer l'acétate d'éthyle, par réaction de l'éthylène et de l'acide acétique, en phase vapeur, ne se sont pas révélées satisfaisantes, dans le cadre d'une telle synthèse, à l'échelle industrielle.

Or, il est bien connu que l'acétate d'éthyle est l'un des esters les plus utilisés dans l'industrie, en raison, notamment, de ses qualités exceptionnelles de solvant. C'est pourquoi il serait fort souhaitable de pouvoir disposer de catalyseurs à la fois plus efficaces et plus stables, dans les conditions requises pour la préparation de cet ester, par le procédé faisant intervenir la réaction de l'éthylène sur l'acide acétique, méthode dont l'intérêt potentiel a déjà été largement reconnu.

La présente invention a donc pour objet un procédé de préparation de l'acetate d'éthyle par mise en contact d'éthylène et d'acide acétique, en phase vapeur, caractérisé en ce que l'on opère en présence d'un polymère solide fluoré portant des groupements acides sulfoniques pendants, et contenant, de préférence des unités structurales de formules I, II ou III, telles que définies ci-après:

$$-CF_2-\underset{\displaystyle SO_3H}{\underset{|}{CF}}- \qquad (I)$$

$$\text{ou}\quad F-\underset{\displaystyle \underset{|}{CF_2}}{\underset{|}{\overset{|}{C}}}-[X]_n-OCF_2CFRSO_3H \qquad (II)$$

$$\text{ou}\quad F-\underset{\displaystyle \underset{|}{CF_2}}{\underset{|}{\overset{|}{C}}}-[X]_n-O\underset{\displaystyle SO_3H}{\underset{|}{C}}F-CF_2R \qquad (III)$$

dans lesquelles

n est un entier compris entre 1 et 5 inclus,

R représente un atome de fluor, ou un radical monovalent perfluoroalcoyle ayant, de préférence, de 1 à 10 atomes de carbone,

et

X représente l'un des groupes $O(CF_2)_m$, $OCF_2CFY$, ou $OCFYCF_2$,

m étant un entier supérieur ou égal à 2 et inférieur ou égal à 10.

Y étant un atome fluor ou un groupement trifluorométhyle.

De tels catalyseurs sont des polymères fluorés ayant des groupes acides à raison d'environ 0,01 à 6 milliéquivalents par gramme de catalyseur. Ces polymères contiennent de préférence, environ de 0,05 à 2 meq/g de groupes acides.

Les catalyseurs contenant l'unité structurale (I) précédemment définie sont des polymères des acides perfluoroalkenyles sulfoniques ou des copolymères de ces acides avec des fluoroéthylènes, tel que le tétrafluoroéthylene. Ces polymères peuvent être préparés par les méthodes décrites dans les brevets ETATS UNIS d'AMERIQUE n° 3 041 317 et 3 624 053.

Lest catalyseurs contenant l'unité structurale (II) ou (III), précédemment définie, peuvent être préparés par diverses méthodes connues, dont les détails figurent dans les brevets des Etats Unis d'Amérique n° 3 282 875 et 3 882 093.

Parmi ces catalyseurs, on préfère les polymères contenant l'unité structurale (II) ou (III) dans laquelle:

n est égale à 1,2 ou 3.

Y est un radical trifluorméthyle

R représente un atome de fluor

et m est égal à 2.

Une classe particulièrement avantageuse de tels polymères est constituée par les copolymères issus de la polymérisation de perfluoroéthylène avec un éther perfluorovinylique — ledit éther comportant des groupements sulfoniques — et correspondant à la structure suivante:

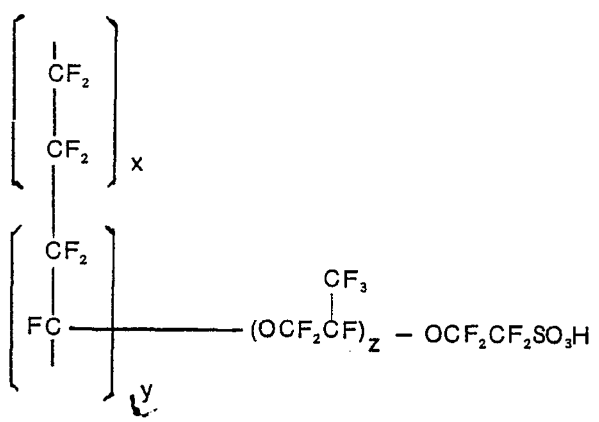

(IV)

dans laquelle z est un entier pouvant varier de 1 à 3 et le rapport x sur y de 2 à 50, ce rapport variant de préférence de 5 à 13.

De tels polymères sont des résines commercialisées sous la marque NAFION.

Outre leur disponibilitié dans le commerce, ces résines présentent l'avantage d'offrir une haute concentration de sites acides accessibles dans une phase solide.

Dans ce qui suit, on appellera résine ou catalyseur les polymères fluorés ayant des groupements sulfoniques, que leur structure soit de type I, II, III ou IV.

Le procédé selon l'invention, présente sur les méthodes antérieurement proposées des avantages surprenants.

En effet, le catalyseur utilisé est très efficace et son activité pratiquement constante sur un grand intervalle de temps. En outre, on n'observe pratiquement pas de formation de sous-produits.

En général, on peut mettre en oeuvre le procédé selon l'invention en introduisant de l'éthylène et de l'acide acétique l'état gazeux dans un zône réactionnelle dans laquelle le catalyseur se trouve disposé sous forme d'une couche solide. Le temps de contact devra être suffisant pour assurer le bon déroulement de la réaction. On opère à la pression atmosphérique, bien que des pressions plus élevées puissent être appliquées sans difficulté.

D'une manière générale la température de réaction est comprise entre 100 et 200°C, des températures légèrement inférieurs ou supérieures pouvant être aussi appliquées. On opére, de préférence, dans la gamme de températures comprises entre 125 et 170°C. Le rapport molaire de l'éthylène à l'acide acétique se situe généralement entre 1 et 30; et de préférence entre 5 et 15. De préférence, le milieu réactionnel est sensiblement anhydre.

De manière générale, le procédé selon l'invention est mis en oeuvre en continu. Les réactifs peuvent être mis en contact avec le catalyseur, par exemple, dans un réacteur tubulaire vertical qui contient, outre une couche centrale de catalyseur, des moyens pour soutenir la couche de catalyseur et des moyens destinés à empêcher l'entraînement du catalyseur solide. On peut par exemple placer la couche de catalyseur sur une grille métallique, ou entre une couche supérieure et une couche inférieure de billes de verre ou de céramique, ou de laine de verre.

Un mode préféré de mise en oeuvre du procédé selon l'invention consiste à mélanger le catalyseur solide avec un matérieur inerte, tel que le quartz ou la silice.

Les exemples ci-après illustrent le procédé selon l'invention.

Exemples 1 à 5

*Préparation du catalyseur*

Le catalyseur utilisé dans les exemples ci-après e été préparé à partir de poudre de NAFION 501, qui est un polymère fluoré du commerce, se présentant sous la forme de sel de potassium. De la poudre de NAFION 501 est traitée avec de l'acide chlorhydrique à 5% (pour convertir le polymère de sa forme $K^+$ en sa forme acide, $H^+$), rincée jusqu'à neutralité avec de l'eau distillée, puis séchée à 100°C sous vide.

*Mode opératoire mis en oeuvre*

Dans un réacteur tubulaire vertical en verre de 67 cm de longeur et d'une capacité de 90 cm3, muni d'une double enveloppe, on charge 50 cm3 de catalyseur éventuellement en mélange avec un matériau inerte, puis dans la partie supérieure du réacteur des billes de verre.

Le réacteur est chauffé par un fluide circulant dans la double enveloppe et il est alimenté par un courant descendant d'éthylène et d'acide acétique. L'effluent est analysé en continu par chromatographie en phase gazeuse.

Le matériau inerte utilisé, le cas échéant est de la silice de surface spécifique 400 m2/g, de diamètre moyen de pore de 80 Å et de volume poreux de 1 cm3/g. Un tel matériau est disponible dans le commerce sous le nom de SPHEROSIL XOA 400.

Le tableau ci-après rassemble les conditions opératoires particulières ainsi que les résultats obtenus.

Dans ce tableau:

. T° C: désigne la température en degrés Celsius.

. t désigne la durée de réaction exprimée en heures

. TTo% désigne le taux de transformation initial de l'acide acétique.

. R désigne le nombre de moles d'acétate d'éthyle formé par kilogramme de catalyseur et par heure, correspondant au taux de transformation initial.

| N° EXEMPLE | POIDS DE CATALYSEUR EN g | POIDS DE SILICE EN g | $C_2H_4$ l/h | $CH_3COOH$ g/h. | T°C | t | TTo % | R |
|---|---|---|---|---|---|---|---|---|
| 1 | 44.7 | 0 | 5.6 | 1.3 | 126 | 65 | 30.0 | 0.141 |
| 2 | 30 0 | 15.0 | 9.0 | 1.9 | 135 | 100 | 59.0 | 0.621 |
| 3 | 20.0 | 15.0 | ,, | 1.8 | ,, | 42 | 46.0 | 0.69 |
| 4 | ,, | ,, | ,, | ,, | 150 | 30 | 60.0 | 0.90 |
| 5 | 15.0 | 15.7 | ,, | 2.0 | ,, | 24 | 58.0 | 1.276 |

Exemple 6

Dans les conditions de l'Exemple 2 ci-avant, on a déterminé le taux de transformation de l'acide acétique et le rendement en acétate d'éthyle au bout de 100 heures de réaction.

On obtient 48% de taux de transformation, et 0,505 moles d'acétate d'éthyle par kilogramme de catalyseur et par heure.

Cet exemple met en évidence la durée de vie du catalyseur:l'activité du catalyseur n'a pratiquement pas diminué au boute de 100 heures de fonctionnement.

**Revendications**

1. Procédé de fabrication de l'acétate d'éthyle par réaction de l'éthylène sur l'acide acétique en phase vapeur, caractérisé en ce que l'on opère en présence d'un polymère fluoré solide contenant des groupements acides sulfoniques pendants.

2. Procédé de fabrication de l'acétate d'éthyle par réaction de l'éthylène sur l'acide acétique en phase vapeur, caractérisé en ce que l'on opère en présence d'un polymère contenant des unités structurales choisies dans le groupe constitué par les unités de formule I, II et III.

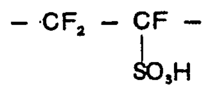 (I)

ou 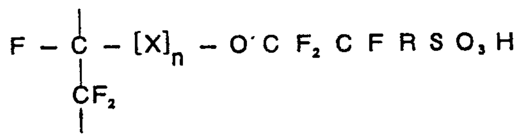 (II)

ou 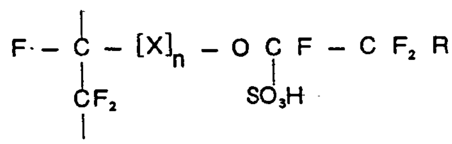 (III)

dans lesquelles

n est un entier compris entre 1 et 5 inclus,

R représente un atome de fluor, ou un radical monovalent perfluoroalcoyle ayant, de préférence, de 1 à 10 atomes de carbone,

et

X représente l'un des groupes $O(CF_2)_m$, $OCF_2CFY$, ou $OCFYCF_2$

m étant un entier supérieur ou égal à 2 et inférieur ou égal à 10.

Y étant un atome fluor ou un groupement trifluorométhyle.

3. Procédé selon la revendication 2, caractérisé en ce que le catalyseur contient des unités structurales choisies dans le groupe constitué par les unités de formuler II et III,

dans lesquelles

n est égal à 1, 2 ou 3

Y est radical trifluorométhyle

R est une atome de fluor

et

m est égal à 2.

4. Procédé de fabrication de l'acétate d'éthyle par réaction de l'éthylène sur l'acide acétique en phase vapeur, caractérisé en ce que l'on opère en présence d'un polymère contenant des unités structurales de formule

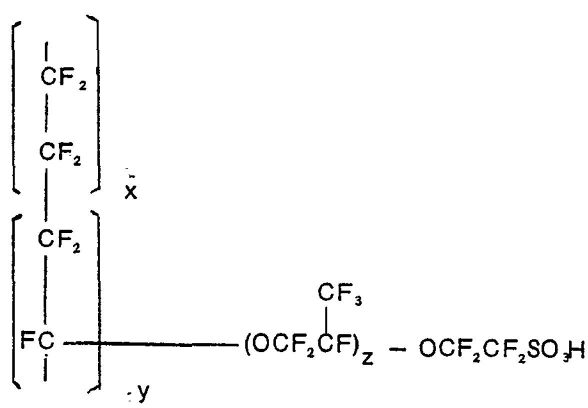 (IV)

dans laquelle z est un entier pouvant varier de 1 à 3 et le rapport x/y de 2 à 50, ce rapport variant de préférence de 5 à 13.

5. Procédé selon l'une quelconque des revendications précédentes, caractérisé en ce que les polymères utilisés contiennent environ de 0,01 à 6 meq acides/g, et de préférence environ de 0,05 à 2 meq acides/g.

6. Procédé selon l'une quelconque des revendications précédentes, caractérisé en ce que le polymère est mis en oeuvre sous la forme d'un mélange avec un matériau inerte, de préférence de la silice.

7. Procédé selon l'une quelconque des revendications précédentes, caractérisé en ce que l'on opère à pression atmosphérique.

8. Procédé selon l'une quelconque des revendications précédentes caractérisé en ce que l'on opère à une température comprise entre 100 et 200°C, et de préférence entre 125 et 170°C.

9. Procédé selon l'une quelconque des revendications précédentes, caractérisé en ce que le rapport molaire de l'éthylène à l'acide acétique est compris entre 1 et 30, et de préférence entre 5 et 15.

**Claims**

1. Process for the manufacture of ethyl acetate by reacting ethylene with acetic acid, in the vapour phase, characterised in that the reaction is carried out in the presence of a solid fluorine-containing polymer in which pendent sulphonic acid groups are present.

2. Process for the manufacture of ethyl acetate by reacting ethylene with acetic acid, in the vapour phase, characterised in that the reaction is carried out in the presence of a polymer containing structural units chosen from the group comprising the units of the formulae I, II and III:

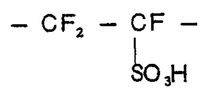
(I)

or 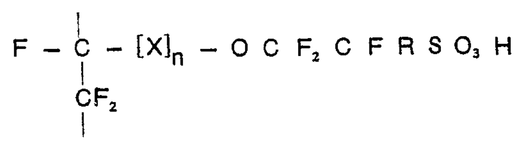
(II)

or 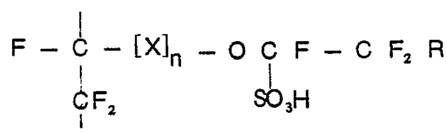
(III)

in which n is an integer between 1 and 5 inclusive, R represents a fluorine atom or a monovalent perfluoroalkyl radical preferably having from 1 to 10 carbon atoms and X represents one of the groups $O(CF_2)_m$, $OCF_2CFY$ or $OCFYCF_2$, m being an integer greater than or equal to 2 and less than or equal to 10 and Y being a fluorine atom or a trifluoromethyl group.

3. Process according to claim 2, characterised in that the catalyst contains structural units chosen from the group comprising the units of formulae II and III in which n is equal to 1, 2 or 3, Y is a trifluoromethyl radical, R is a fluorine atom and m is equal to 2.

4. Process for the manufacture of ethyl acetate by reacting ethylene with acetic acid, in the vapour phase, characterised in that the reaction is carried out in the presence of a polymer containing structural units of the formula:

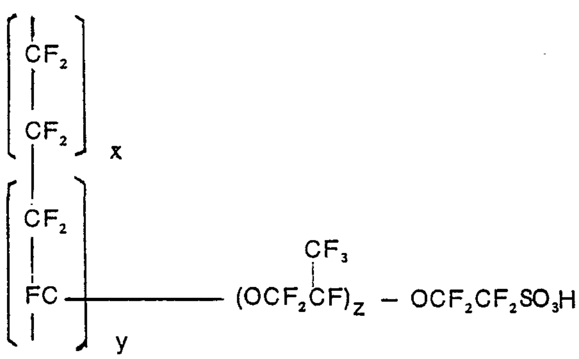
(IV)

in which z is an integer which can vary from 1 to 3 and the ratio x/y can vary from 2 to 50, this ratio preferably varying from 5 to 13.

5. Process according to any one of the preceding claims, characterised in that the polymers used contain from about 0.01 to 6 milliequivalents of acid/g and preferably from about 0.05 to 2 milliequivalents of acid/g.

6., Process according to any one of the preceding claims, characterised in that the polymer is employed in the form of a mixture with an inert material, preferably silica.

7. Process according to any one of the preceding claims, characterised in that the reaction is carried out at atmospheric pressure.

8. Process according to any one of the preceding claims, characterised in that the reaction is carried out at a temperature between 100 and 200°C and preferably between 125 and 170°C.

9. Process according to any one of the preceding claims, characterised in that the molar ratio of ethylene to acetic acid is between 1 and 30 and preferably between 5 and 15.

**Patentansprüche**

1. Verfahren zur Herstellung von Äthylacetat durch Umsetzung von Äthylen mit Essigsäure in der Dampfphase, dadurch gekennzeichnet, daß man in Gegenwart eines festen fluorhaltigen Polymerisats arbeitet, das hängende Sulfonsäuregruppen enthält.

2. Verfahren zur Herstellung von Äthylacetat durch Umsetzung von Äthylen mit Essigsäure in der Dampfphase, dadurch gekennzeichnet, daß man in Gegenwart eines Polymerisats arbeitet, das Struktureinheiten enthält die ausgewählt sind aus der Gruppe bestehend aus Einheiten der Formeln I, II und III

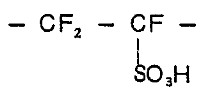

(I)

oder 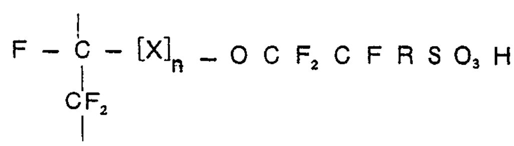

(II)

oder 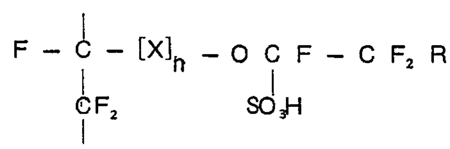

(III)

in denen

n eine ganze Zahl von 1 bis 5 ist,

R ein Fluoratom oder eine einwertige Perfluoralkylgruppe mit vorzugsweise 1 bis 10 Kohlenstoffatomen bedeutet und

X für eine der Gruppen $O(CF_2)_m$, $OCF_2CFY$ oder $OCFYCF_2$ steht

m eine ganze Zahl gleich oder größer 1 und gleich oder kleiner 10 ist,

Y ein Fluoratom oder eine Trifluoromethylgruppe bedeutet.

3. Verfahren nach Anspruch 2, dadurch gekennzeichnet, daß der Katalysator Struktureinheiten enthält, die ausgewählt sind aus der Gruppe bestehend aus Einheiten der Formeln II und III, in denen

n 1, 2 oder 3 ist,

Y die Trifluormethylgruppe bedeutet,

R ein Fluoratom is und

m gleich 2 ist.

4. Verfahren zur Herstellung von Äthylacetat durch Umsetzung von Äthylen mit Essigsäure in der Dampfphase, dadurch gekennzeichnet, daß man in Gegenwart eines Polymerisats arbeitet, welches Struktureinheiten der Formel

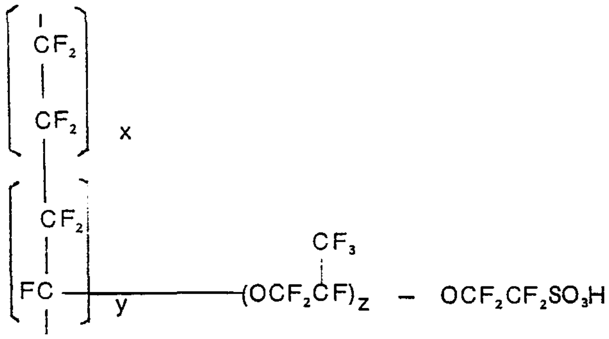

(IV)

enthält, in der z eine ganze Zahl von 1 bis 3 ist und das Verhältnis x/y 2 bis 50 beträgt, wobei diseses Verhältnis vorzugsweise im Bereich von 5 bis 13 liegt.

5. Verfahren nach einem der vorangegangenen Ansprüche, dadurch gekennzeichnet, daß die verwendeten Polymerisate etwa 0,01 bis 6 mÄq. Säuregruppen/g und vorzugsweise etwa 0,05 bis 2 mÄq. Säuregruppen/g enthalten.

6. Verfahren nach einem der vorangegangenen Ansprüche, dadurch gekennzeichnet, daß das Polymerisat in Form eines Gemisches mit einem inerten Material, vorzugsweise mit Kieselsäure eingesetzt wird.

7. Verfahren nach einem der vorangegangen Ansprüche, dadurch gekennzeichnet, daß man bei Atmosphärendruck arbeitet.

8. Verfahren nach einem der vorangegangenen Ansprüche, dadurch gekennzeichnet, daß man bei einer Temperatur im Bereich von 100 bis 200°C und vorzugsweise im Bereich von 125 bis 170°C arbeitet.

9. Verfahren nach einem der vorangegangenen Ansprüche, dadurch gekennzeichnet, daß das Molverhältnis von Äthylen zu Essigsäure 1 bis 30 und vorzugsweise 5 bis 15 beträgt.